# EUROPEAN PATENT SPECIFICATION

(11) **EP 1 441 680 B1**
(45) Date of publication and mention of the grant of the patent: **26.12.2012**
(21) Application number: 02760946.0
(22) Date of filing: 15.08.2002
(51) Int. Cl.: A61F 13/56, A61F 13/49, A61F 13/64

(54) **ABSORBENT ARTICLE**
SAUGFÄHIGER ARTIKEL
ARTICLE ABSORBANT

(30) Priority: 22.08.2001 SE 0102805
(43) Date of publication of application: 04.08.2004
(73) Proprietor: SCA Hygiene Products AB, 405 03 Göteborg (SE)
(72) Inventor: ALMBERG, Christian, S-435 41 Mölnlycke (SE)
(74) Representative: Egeröd, Lisbeth
(86) International application number: PCT/SE2002/001455
(87) International publication number: WO 2003/017902

(56) References cited:
- EP-A1- 0 291 984
- WO-A1-00/20206

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a pair of belt members attached to the rear portion , alternatively to the front portion, of the article and which are intended to be fastened together around the waist of the wearer and where said front portion, alternatively said rear portion, is provided with fastening means intended to be fastened to the belt members, in such a way that the article will assume a pantlike shape, where the belt members form a part of the waist portions of the pant.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other. It is further known, through e g EP-A-0 287 388, EP-A-0 409 307, EP-A-0 605 012 and FR-A-2 586 558, to attach the front and rear portions of the article by means of a belt. The belt members are usually attached to the rear portion of the diaper and are intended to be fastened together around the waist of the wearer and fastening means provided at the front portion of the diaper are then intended to be fastened to the outside of the belt members. The belt provides improved possibilities to adjust the fit of the diaper. The belt further provides a simplified change of diaper or incontinence guard, especially when the wearer is standing.

One problem with these belts is that they may cause skin irritations to the user, due to that the belt is in direct contact with the skin of the wearer and has to be tightened relatively strongly in order to have a satisfactory fit and security against leakage of the diaper or incontinence guard. By the tight contact and friction between the belt and the skin there will be a mechanic wear of the skin which gives rise to irritation and even skin injuries. It is therefor important that the material used to form the inside of the belt is soft and skin-friendly. Belt materials dealing with this problem are disclosed in WO 00/27330 and in WO 01/00129.

Another problem that may occur with belts is that they may tear when tightened around the wearer. This problem especially occurs for belts which are wholly made of fibrous material, often two or more layers of nonwoven materials bonded together into a laminate. Belts in which one layer is a plastic film are often strong, but they have a drawback of being tight and relatively stiff and therefor not so comfortable to wear.

### Object and most important features of the invention

An object of the present invention is to provide a belt for absorbent articles which is comfortable to wear and which is resistant to tearing. This has according to the invention been provided by the fact that the belt members comprises a flexible laminate of at least two layers of fibrous material bonded together in a bonding pattern provided by ultrasonic or laser welding and/or heat calendering, said bonding pattern having a bonding area of no more than 10% , and said laminate having a tear strength of at least 22 N.

According to one embodiment said laminate has a tear strength of at least 24N, preferably at least 25 N and more preferably at least 30 N.

According to further preferred embodiments the bonding pattern has a bonding area of no more than 8% and preferably no more than 5%

In a further aspect of the invention the bonding pattern has a tightness of bonding sites of between 1 and 15 bonding sites per cm² and preferably between 1 and 10 bonding sites per cm².

According to one embodiment said laminate comprises at least three layers of fibrous material, one outer layer acting as a loop material for a hook-and-loop type fastener, a middle layer of a relatively tear strong fibrous material and an inner layer of a soft and skin friendly fibrous material.

An example of a relatively tear strong fibrous material suited for said middle layer is a nonwoven material comprising continuous filaments, such as a spunbond and/or meltblown material.

According to one embodiment of the invention said outer layer acting as an attachment surface for said fastening means, especially as a loop material for a hook-and-loop type fastener, is creped.

### Description of drawings

The invention will in the following be closer described with reference to an embodiment shown in the accompanying drawings.
Fig. 1 shows a schematic perspective view of a belt-provided diaper according to the invention.
Fig. 2 shows schematically a broken view of a laminate according to the invention.

### Description of embodiments

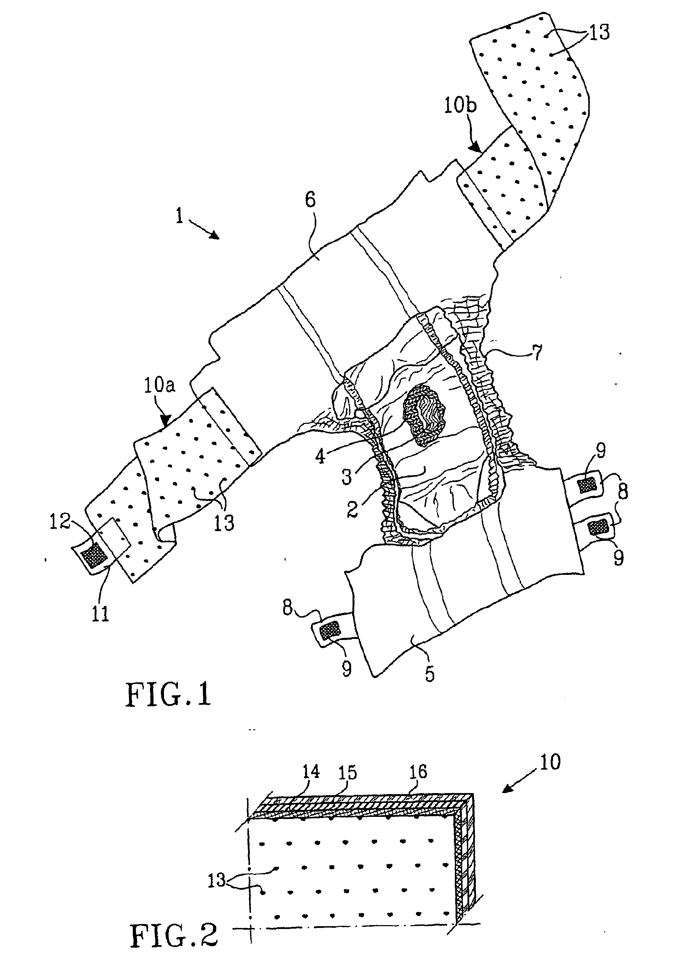

Fig. 1 of the drawings shows an embodiment of a diaper or incontinence guard 1 comprising a liquid permeable topsheet 2, a liquid impermeable backsheet 3 and an absorbent body 4 enclosed therebetween. The liquid permeable topsheet 2 can be any material used for this purpose, for example a nonwoven material, such as a spunbond material of continuous filaments, a meltblown material, a thermobonded fibrous web such as a carded fibrous web. The topsheet may also be a layer of so called tow fibers bonded in a bonding pattern or a perforated plastic film.

The liquid impermeable backsheet 3 may also be any material used for this purpose, such as a plastic film, a nonwoven material coated with a liquid impervious material, a hydrophobic nonwoven material which resists liquid penetration and/or a laminate of plastic film and nonwoven material. Breathable materials which are permeable to air and water vapour but which resist liquid penetration at least up to a certain pressure may also be used as backsheet materials.

The topsheet 2 and the backsheet material 3 have a somewhat greater extension in the plane than the absorbent body 4 and extends outside the edges thereof. The layers 2 and 3 are connected to each other within the projecting portions thereof, e g by gluing or welding by heat or ultrasonic.

The absorbent body 4 can be of any kind used for this purpose. Examples of commonly used absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provided with a pair of tabs 8 carrying attachment means such as a hook material 9 of a so called hook-and-loop type fastener. or other type of attachment means such as adhesive tape.

The term "hook material" is used to designate the portion of a mechanical fastening means having engaging "hook" element. However it is not intended to limit the shape of the engaging elements to include only "hooks" but encompasses any shape of engaging elements, unidirectional or bidirectional, known in the art to mechanically engage a complementary loop fastening material.

A pair of belt members 10a and b are with one end attached, e g glued or ultrasonically welded to the rear part 6 of the diaper. The belt members 10a,b are with their opposite ends intended to be fastened together, e g by a tab 11 on one belt member 10a, said tab carrying a hook material 12 of a so called hook-and-loop type fastener. The hook material 12 on the tab 11 is intended to attach to the outside of the opposite belt member 10b. Instead of hook-and-loop type fastener 12 there may be another type of optional attachment means, such as adhesive tape.

The tabs 8 carrying a hook material or corresponding attachment means of the front portion 5 are intended to be attached to the outside of the belt members 10a,b in order to fasten together the diaper to the desired pantlike shape.

In an alternative embodiment the belt members 10 a, b are attached to the front portion 5 of the diaper and are then fastened together on the back of the wearer. Tabs 8 carrying fastening means, for example a hook material or an adhesive tape, are in this case provided at the rear portion 6 of the diaper.

The outside of the belt members 10a,b should act as a reception surface cooperating with the fastening means on tabs 8 and 11. For hook-and-loop fasteners the material on the outside of the belt portions should serve as a loop material. The term "loop" in this respect is not limited only to materials in which discrete, separately formed loops of material are adapted to receive and engage the hook elements of a complementary hook material, but the loop material also includes fibrous nonwoven in which the individual fibers function to engage the hook elements without such fibers being formed into discrete loops.

For tape fasteners the material on the outside of the belt members 10 a,b should serve as attachment surface for adhesive tapes. Certain nonwoven materials will function both as loop material for hook-and-loop fasteners and as attachments surface admitting refastening of an adhesive tape. This is disclosed in WO 01/00129.

The width of the belt members should be between 5 and 20 cm, preferably between 7 and 15 cm.

The belt members according to this invention comprise a flexible laminate of at least two layers of fibrous material bonded together in a bonding pattern 13 provided by ultrasonic, laser and/or heat. At least some of the fibers in the layers of fibrous material should therefore be meltable by such bonding techniques. In one embodiment disclosed in Fig. 2 the laminate comprises three layers 14, 15 and 16. The bonding pattern 13 should have a bonding area of no more than 10% and the laminate should have a tear strength of at least 22 N. This will make the belt members resist tearing as the belt is tightened around the waist of the wearer. Tests have proven that the tearing frequency at normal use for belts having a tear strength of 21 N and lower was unaccetably high. Preferably the tear strength should be at least 24 N, more preferably at least 25 N and most preferably at least 27N. For those belt having a tear strength of 29 N or higher there were no tearing at all.

### Tear strength

The tear strength is measured by EDANA test method TEAR 70.3-96 with the modification that a conditioning time of at least 4h, a temperature of 23°C and a relative humidity of 50% R.H. is used.

### Bonding area and bonding tightness

A bonding area of more than 10% will result in an increased amount of tearing indications or notches and an increased risk for tearing of the belt members.

Preferably the boding area should be no more than 8% and more preferably no more than 5%.

The bonding pattern comprises a plurality of bonding sites in the form of points, lines, spots or the like arranged in a pattern. The bonding area of a bonding pattern is defined as the amount of the pattern that consists of the bonding sites.

Another important factor for providing high tear strength is the bonding tightness, which is the number of bonding sites per area unit. It is preferred that the bonding pattern 13 has a bonding tightness of between 1 and 15 bonding sites per cm². Preferably it has a bonding tightness of between 1 and 10 bonding sites per cm². With a high bonding tightness more tearing indications or notches are formed, which will deteriorate the tearing strength.

It is pointed out that by relatively large bonding sites, for example in the form of lines, a relatively large bonding area may be provided with a relatively small number of bonding sites, as compared to a bonding pattern of small bonding sites, for example in the form of points, which have to arranged tighter in order to provide the same bonding area as for a pattern of larger bonding size. Thus both bonding area and bonding tightness are important.

### Preferred example of a laminate material

One embodiment of a laminate according to the invention is a nonwoven laminate of at least three fibrous material layers 14, 15 and 16. One outer layer 14 intended to form the outside of the belt is a fibrous material adapted to serve as an attachment surface for the fastening means on the tabs 8 and 11. Examples of nonwoven materials are spunbond, meltblown, carded bonded materials etc. The middle layer 15 should be of a relatively tear strong fibrous material, such as a spunbond or meltblown material comprising continuous filaments. The other outer layer 16, intended to form an inner layer of the belt facing the wearer, should be of a soft and skin friendly fibrous material. Examples of suitable materials are spunbond and meltblown materials, carded bonded materials etc. Examples of polymer materials used in the different fibrous materials may be any suited for this purpose, for ecample polypropylene, polyethylene, polyester and /or so called bicomponent fibers.

The basis weight of the nonwoven laminate can vary between 40 and 150 gsm, preferably between 60 and 120 gsm and more preferably between 75 and 105 gsm.

One or more layers of the laminate may be creped. According to one embodiment the outer layer 14 intended to act as receiving material for the fastening means, especially as a loop material for a hook-and-loop type fastener, is creped. By the creping the loop function of the material is improved.

One non-limiting example of a laminate according to the invention is a three-layered laminate:
Carded thermobonded material, basis weight 30 gsm, PP fibers of 2.2 dtex;
Spunbond layer, basis weight 40 gsm, PP fibers of 2.2 dtex;
Carded thermobonded material, basis weight 22 gsm, PP fibers of 2.2 dtex.

The spunbond layer is used as the middle layer, the carded material having the highest basis weight is used as outside of belt and is adapted to act as loop material for a hook-and-loop type fastener and the carded material having the lowest basis weight is used as inner skin-facing side of the belt.

The laminate is bonded by ultrasonic bonding with a bonding area of about 3% and a bonding tightness of about 7 bonding sites per cm². The tear strength is between 50 and 60 N.

## Claims

1. Absorbent article such as a diaper and an incontinence guard comprising a liquid permeable topsheet (2), a liquid impermeable backsheet (3) and an absorbent body (4) enclosed therebetween, said article having a front portion (5), a rear portion (6) and a crotch portion (7) therebetween, and further is provided with a pair of belt members (10 a,b) attached to the rear portion (6), alternatively to the front portion, of the article and which are intended to be fastened together around the waist of the wearer by fastening means (11,12) and where said front portion (5), alternatively said rear portion, is provided with fastening means (8,9) intended to be fastened to the belt members (10 a,b), in such a way that the article will assume a pantlike shape, where the belt members (10 a,b) form a part of the waist portions of the pant,
**characterized in**
**that** the belt members (10 a,b) comprise a flexible laminate of at least two layers of fibrous material bonded together in a bonding pattern (13) provided by ultrasonic, laser and/or heat, said bonding pattern having a bonding area of no more than 10% , and said laminate having a tear strength of at least 22 N as measured according to the method specified in the specification.

2. Absorbent article as claimed in claim 1,
**characterized in**
**that** said laminate has a tear strength of at least 24N.

3. Absorbent article as claimed in claim 1,
**characterized in**
**that** said laminate has a tear strength of at least 25N.

4. Absorbent article as claimed in claim 1,
**characterized in**
**that** said laminate has a tear strength of at least 30N.

5. Absorbent article as claimed in any of claims 1-4,
**characterized in**
**that** said bonding pattern having a bonding area of no more than 8%.

6. Absorbent article as claimed in any of claims 1-4,
**characterized in**
**that** said bonding pattern having a bonding area of no more than 5%.

7. Absorbent article as claimed in any of claims 1-6,
**characterized in**
**that** said bonding pattern (13) has a tightness ofbonding sites of between 1 and 15 bonding sites per cm².

8. Absorbent article as claimed in claim 7,
**characterized in**
**that** said bonding pattern (13) has a tightness of bonding sites of between 1 and 10 bonding sites per cm².

9. Absorbent article as claimed in any of claims 1-8,
**characterized in**
**that** said laminate comprises at least three layers of fibrous material, one outer layer (14) acting as an attachments surface for said fastening means (8,9;11,12), a middle layer (15) of a relatively tear strong fibrous material and an inner layer (16) of a soft and skin friendly fibrous material.

10. Absorbent article as claimed in claim 9,
**characterized in**
**that** said middle layer (15) is layer comprising continuous filaments, such as spunbond and meltblown material.

11. Absorbent article as claimed in claim 9 or 10,
**characterized in**
**that** said outer layer (14) acting as an attachments surface for said fastening means (8,9; 11,12), especially as a loop material for a hook-and-loop type fastener, is creped.

## Patentansprüche

1. Saugfähiger Artikel, wie zum Beispiel eine Windel und ein Inkontinenzschutz, mit einer flüssigkeitsdurchlässigen Oberschicht (2), einer flüssigkeitsundurchlässigen Rückschicht (3) und einem dazwischen liegenden saugfähigen Körper (4), wobei der Artikel einen vorderen Abschnitt (5), einen hinteren Abschnitt (6) und dazwischen einen Schrittabschnitt (7) aufweist, und des Weiteren mit einem Paar Gürtelelemente (10a, b) bereitgestellt ist, das an dem hinteren Abschnitt (6) oder alternativ an dem vorderen Abschnitt des Artikels angebracht ist und das vorgesehen ist, über ein Befestigungsmittel (11, 12) um die Taille des Trägers aneinander befestigt zu werden, und wobei der vordere Abschnitt (5) oder alternativ der hintere Abschnitt mit einem Befestigungsmittel (8, 9) bereitgestellt ist, das vorgesehen ist, so an den Gürtelelementen (10a, b) befestigt zu werden, dass der Artikel eine hosenartige Form annimmt, wobei die Gürtelelemente (10a, b) einen Teil des Taillenabschnitts der Hose ausbilden,
**dadurch gekennzeichnet,**
**dass** die Gürtelelemente (10a, b) ein flexibles Laminat aus mindestens zwei Lagen fasrigen Materials aufweisen, die mit einem Verbindungsmuster (13) miteinander verbunden sind, das durch Ultraschall, Laser und/oder Wärme bereitgestellt ist, wobei das Verbindungsmuster eine Verbindungsfläche von nicht mehr als 10 % und das Laminat eine Zugfestigkeit von mindestens 22 N aufweist, und zwar gemessen nach dem in der Beschreibung spezifizierten Verfahren.

2. Saugfähiger Artikel nach Anspruch 1,
**dadurch gekennzeichnet,**
**dass** das Laminat eine Reißfestigkeit von mindestens 24 N aufweist.

3. Saugfähiger Artikel nach Anspruch 1,
**dadurch gekennzeichnet,**
**dass** das Laminat eine Reißfestigkeit von mindestens 25 N aufweist.

4. Saugfähiger Artikel nach Anspruch 1,
**dadurch gekennzeichnet,**
**dass** das Laminat eine Reißfestigkeit von mindestens 30 N aufweist.

5. Saugfähiger Artikel nach einem der Ansprüche 1-4,
**dadurch gekennzeichnet,**
**dass** das Verbindungsmuster eine Verbindungsfläche von nicht mehr als 8 % aufweist.

6. Saugfähiger Artikel nach einem der Ansprüche 1-4,
**dadurch gekennzeichnet,**
**dass** das Verbindungsmuster eine Verbindungsfläche von nicht mehr als 5 % aufweist.

7. Saugfähiger Artikel nach einem der Ansprüche 1-6,
**dadurch gekennzeichnet**,
das das Verbindungsmuster (13) eine Verbindungsstellendichte zwischen 1 und 15 Verbindungsstellen pro cm² aufweist.

8. Saugfähiger Artikel nach Anspruch 7,
**dadurch gekennzeichnet,**
**dass** das Verbindungsmuster (13) eine Verbindungsstellendichte zwischen 1 und 10 Verbindungsstellen pro cm² aufweist.

9. Saugfähiger Artikel nach einem der Ansprüche 1-8,
**dadurch gekennzeichnet,**
**dass** das Laminat mindestens drei Lagen fasrigen Materials aufweist, und zwar eine äußere Lage (14), die als eine Befestigungsfläche für das Befestigungsmittel (8, 9; 11, 12) wirkt, eine mittige Lage (15) eines relativ reißfesten fasrigen Materials und eine innere Lage (16) aus einem weichen und hautfreundlichen fasrigen Material.

10. Saugfähiger Artikel nach Anspruch 9,
**dadurch gekennzeichnet,**
**dass** die mittige Lage (15) eine Lage mit durchgängigen Filamenten ist, wie zum Beispiel ein durch ein Spinnvliesverfahren hergestelltes oder heißluftgezogenes Material.

11. Saugfähiger Artikel nach Anspruch 9 oder 10,
**dadurch gekennzeichnet,**
**dass** die äußere Lage (14), die als Befestigungsfläche für das Befestigungsmittel (8, 9; 11, 12) wirkt, insbesondere als Schleifenmaterial für ein haken- und schleifenartiges Befestigungselement, gekreppt ist.

## Revendications

1. Article absorbant tel qu'une couche-culotte et une protection contre l'incontinence comprenant une feuille supérieure (2) perméable aux liquides, une feuille arrière (3) imperméable aux liquides et un corps absorbant (4) enfermé entre celles-ci, ledit article comportant une partie avant (5), une partie arrière (6) et une partie entrejambe (7) située entre celles-ci, et étant muni en outre d'une paire d'éléments ceinture (10 a, b) qui sont fixés à la partie arrière (6), ou optionnellement à la partie avant, de l'article et qui sont destinés à être attachés ensemble autour de la taille de l'utilisateur à l'aide de moyens d'attache (11, 12) et dans lequel article ladite partie avant (5), ou optionnellement ladite partie arrière est munie de moyens d'attache (8, 9) destinés à être attachés aux éléments ceinture (10 a, b) de telle manière que l'article prenne une forme de culotte, les éléments ceinture (10 a, b) constituant une portion des parties taille de la culotte,
**caractérisé en ce que**
les éléments ceinture (10 a, b) comprennent un stratifié souple comportant au moins deux couches de matériau fibreux liées ensemble selon un schéma de liaison (13) exécuté au moyen d'ultrasons, de laser et/ou de chaleur, ledit schéma de liaison présentant une surface de liaison ne dépassant pas 10 % et ledit stratifié étant doté d'une résistance à la déchirure d'au moins 22 N mesurée selon le procédé présenté dans la description.

2. Article absorbant selon la revendication 1
**caractérisé en ce que**
ledit stratifié est doté d'une résistance à la déchirure d'au moins 24 N.

3. Article absorbant selon la revendication 1
**caractérisé en ce que**
ledit stratifié est doté d'une résistance à la déchirure d'au moins 25 N.

4. Article absorbant selon la revendication 1
**caractérisé en ce que**
ledit stratifié est doté d'une résistance à la déchirure d'au moins 30 N.

5. Article absorbant selon l'une quelconque des revendications 1 à 4,
**caractérisé en ce que**
ledit schéma de liaison a une surface de liaison qui ne dépasse pas 8 %.

6. Article absorbant selon l'une quelconque des revendications 1 à 4,
**caractérisé en ce que**
ledit schéma de liaison a une surface de liaison qui ne dépasse pas 5 %.

7. Article absorbant selon l'une quelconque des revendications 1 à 6,
**caractérisé en ce que**
ledit schéma de liaison (13) présente une densité de sites de liaison comprise entre 1 et 15 sites de liaison par cm².

8. Article absorbant selon la revendication 7
**caractérisé en ce que**
ledit schéma de liaison (13) présente une densité de sites de liaison comprise entre 1 et 10 sites de liaison par cm².

9. Article absorbant selon l'une quelconque des revendications 1 à 8,
**caractérisé en ce que**
ledit stratifié comprend au moins trois couches de matériau fibreux, une couche extérieure (14) jouant le rôle de surface de fixation pour lesdits moyens d'attache (8, 9 ; 11, 12), une couche médiane (15) constituée d'un matériau fibreux relativement résistant à la déchirure et une couche intérieure (16) constituée d'un matériau fibreux doux et agréable pour la peau.

10. Article absorbant selon la revendication 9
**caractérisé en ce que**
ladite couche médiane (15) est une couche comprenant des filaments continus tels que ceux d'un matériau soufflé fondu et lié au filage.

11. Article absorbant selon la revendication 9 ou 10
**caractérisé en ce que**
ladite couche extérieure (14) jouant le rôle de surface d'attache pour ledit moyen d'attache (8, 9 ; 11, 12), particulièrement en tant que matériau comportant des boucles destiné à un dispositif d'attache du type à crochets et à boucles, est crêpé.
